# EUROPÄISCHE PATENTSCHRIFT

(11) **EP 0 467 117 B1**
(45) Veröffentlichungstag und Bekanntmachung des Hinweises auf die Patenterteilung: **06.03.1996**
(21) Anmeldenummer: 91110548.4
(22) Anmeldetag: 26.06.1991
(51) Int. Cl.: D01H 13/32, G01B 7/04

(54) **Vorrichtung zum Messen der Dicke und/oder der Ungleichmässigkeit von Vliesen oder Watten an Spinnereivorbereitungsmaschinen**
Device for measuring the thickness and/or the unevenness of fleeces or laps in preparational spinning machines
Dispositif pour mesurer l'épaisseur et/ou l'irrégularité d'une couche de fibres ou d'ouates dans les machines de préparation des métiers à filer

(30) Priorität: 04.07.1990 CH 2219/90
(43) Veröffentlichungstag der Anmeldung: 22.01.1992
(73) Patentinhaber: ZELLWEGER LUWA AG, CH-8610 Uster (CH)
(72) Erfinder: Baechler, François, CH-8615 Wermatswil (CH)

(56) Entgegenhaltungen:
- WO-A-87/05837
- DE-A- 2 544 029
- DE-A- 3 810 575
- US-A- 2 805 449

## Beschreibung

Die vorliegende Erfindung betrifft eine Vorrichtung zum Messen der Dicke und/oder der Ungleichmässigkeit von nachfolgend als Wickel bezeichneten Vliesen oder Watten an Spinnereivorbereitungsmaschinen, mit einer festen Führung für den Wickel und mit einem diesen gegen die Führung drückenden und relativ zu dieser verstellbaren Dickenfühler dessen Verstellweg ein Mass für die Dicke und/oder die Ungleichmässigkeit des Wickels darstellt. Eine solche Vorrichtung ist z.B. aus der DE-A-3 810 575 bekannt.

Bekannte Vorrichtungen dieser Art sind durch eine einzige über die gesamte maximale Wickelbreite reichende Messwalze und durch ein Messorgan zur Messung des Verstellwegs dieser Messwalze gebildet. Ganz abgesehen davon, dass der Verstellweg dieser Messwalze immer nur für den gerade grössten Dickenwert repräsentativ ist, auch wenn dieser nur sehr lokal auftritt, ist diese Vorrichtung auch ausserordentlich träge. Zudem ist sie unflexibel, weil sie nicht an verschiedene Wickelbreiten angepasst werden kann.

Durch die Erfindung soll nun eine Vorrichtung der eingangs genannten Art vorgeschlagen werden, welche eine geringe Trägheit und daher eine hohe Empfindlichkeit aufweist, welche repräsentative Messergebnisse des Dickenverlaufs über die Wickelbreite liefert, und welche an wechselnde Wickelbreiten angepasst werden kann.

Diese Aufgabe wird erfindungsgemäss dadurch gelöst, dass der Dickenfühler durch eine Mehrzahl von über die Breite des Wickels nebeneinander angeordneten und durch die Wickel individuell verstellbaren Einzelfühlern gebildet ist.

Da die Einzelfühler nur einen Bruchteil des Massenträgheitsmoments des bekannten Dickenfühlers aufweisen, wird die Messvorrichtung entsprechend empfindlicher und das bedeutet, dass auch an schnell laufenden Maschinen kurze Variationen der Wickeldicke gemessen werden können. Das Messergebnis jedes Einzelfühlers ist für den entsprechenden streifenförmigen Bereich des Wickels repräsentativ, so dass die Messergebnisse aller Einzelfühler die Dickenverteilung über die Wickelbreite wiedergeben. Schliesslich ermöglicht der Aufbau aus Einzelfühlern eine einfache Anpassung der Messvorrichtung an wechselnde Wickelbreiten.

Nachstehend wird die Erfindung anhand von Ausführungsbeispielen und der Zeichnungen näher erläutert; es zeigen:
- Fig. 1 - 3: je ein Ausführungsbeispiel einer erfindungsgemässen Vorrichtung in Vorderansicht und im Schnitt,
- Fig. 4 - 6: ein Detail der Vorrichtung von Fig. 1 in drei Ansichten,
- Fig. 7: eine Ansicht in Richtung des Pfeiles VII von Fig. 5,
- Fig. 8: eine Ansicht in Richtung des Pfeiles VIII von Fig. 7; und
- Fig. 9, 10: Diagramme zur Funktionserläuterung.

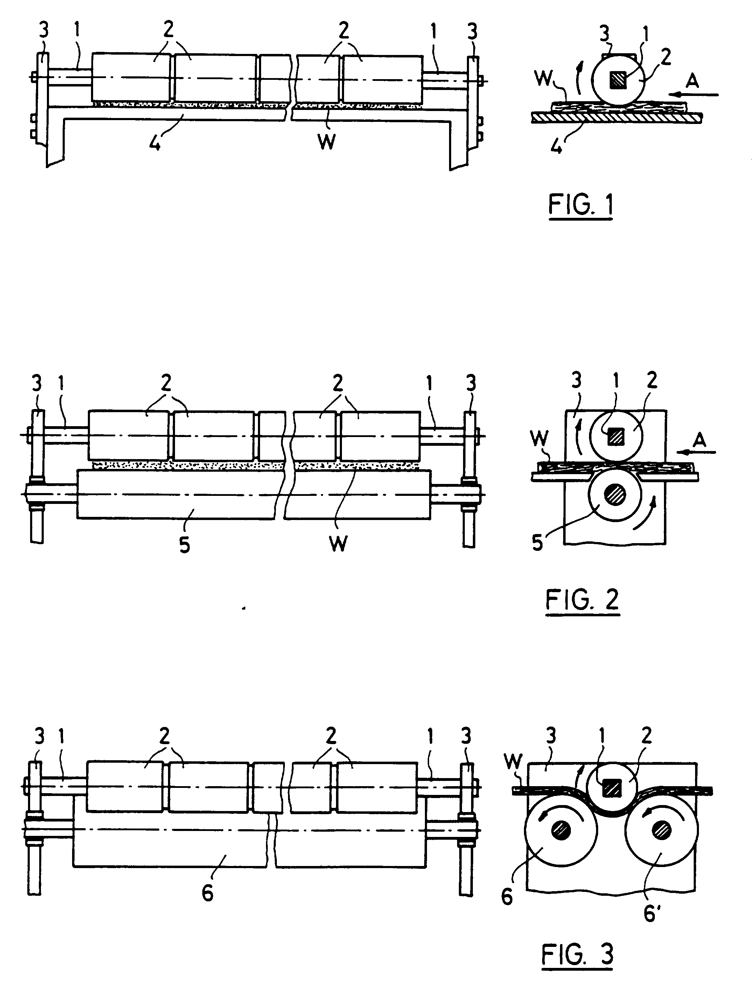

Aus den Fig. 1 bis 3 ist das Prinzip der Messung der Dicke oder der Ungleichmässigkeit von Vliesen oder Watten an Spinnereivorbereitungsmaschinen, wie Karden, Strecken, Kämmaschinen und dergleichen ersichtlich. Diese Messung erfolgt durch Messung des Abstands zwischen einer festen Führung für das Vlies oder die Watte, die nachfolgend als Wickel W bezeichnet werden, und einem den Wickel gegen diese Führung drückenden Dickenfühler, welcher darstellungsgemäss durch eine Mehrzahl von auf einer gemeinsamen Welle 1 gelagerten Messwalzen 2 gebildet ist.

Die Welle 1, welche vorzugsweise durch einen Balken mit beispielsweise rechteckigem Querschnitt gebildet ist, ist in einer relativ zur Führung für den Wickel W verstellbaren Halterung 3 montiert. Diese Halterung ist durch geeignete Tragarme, Lagerböcke oder dergleichen gebildet. Die Anpresskraft der Messwalzen 2 auf den Wickel W wird durch Einstellung des Abstandes zwischen der Welle 1 und der Führung an der oder mittels der Halterung 3 bestimmt.

Die in den Fig. 1 bis 3 dargestellten Ausführungsbeispiele unterscheiden sich nur durch die Ausbildung der Führung für den Wickel W, welche in Fig. 1 durch eine ebene Platte 4, in Fig. 2 durch eine einzelne Walze 5 und in Fig. 3 durch ein Walzenpaar 6, 6' gebildet ist. Die Transportrichtung des Wickels W ist jeweils mit einem Pfeil A bezeichnet, die Führungswalze 5 und mindestens eine der Führungswalzen 6, 6' sind angetrieben. Die Messwalzen 2 sind auf der gemeinsamen Welle 1 frei rotierbar.

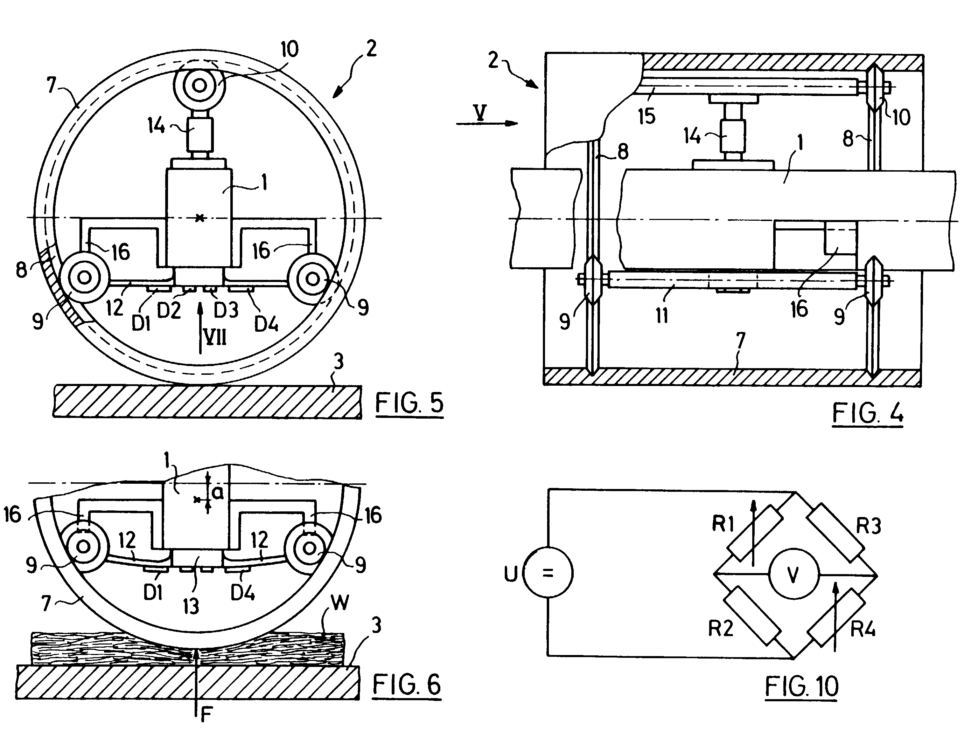

Das gemeinsame wesentliche Merkmal aller Ausführungsbeispiele ist die Mehrzahl der auf der balkenförmigen Welle 1 angeordneten Messwalzen 2, welche jede für sich und unabhängig von den anderen als Fühler für die Dicke des zugeordneten streifenförmigen Bereichs des Wickels W wirkt. Wegen des verglichen mit einer einzigen, über die gesamte Maschinenbreite reichenden Messwalze geringen Massenträgheitsmoments, können die einzelnen Messwalzen 2 auch kurze Variationen der Dicke des Wickels W an schnell laufenden Maschinen messen. Der modulare Aufbau des Dickenfühlers durch die einzelnen Messwalzen 2 ermöglicht eine einfache Anpassung des Messsystems an verschiedene Wickelbreiten, und die Bereichs-Anpassung des Messsystems ist durch Verstellen des Abstandes der Welle 1 von der Führung 4, 5, 6, 6' ebenfalls einfach möglich. Wie anhand der Figuren 4 bis 6 ersichtlich sein wird, ist das gesamte Messsystem ausserdem hebelarm- und spielfrei.

Fig. 4 zeigt einen Axialschnitt durch eine Messwalze 2, die Fig. 5 und 6 eine Ansicht in Richtung des Pfeiles V von Fig. 4 in zwei verschiedenen Schwenkstellungen der Messwalze 2. Darstellungsgemäss sind die Messwalzen 2 durch Hohlzylinder 7 gebildet, welche an ihrer Innenflächen zwei parallele, beabstandete und quer zur Zylinderachse verlaufende Nuten 8 aufweisen, in welchen Rollen 9 und 10 einer dreiachsigen Aufhängung geführt sind. Diese Aufhängung besteht aus einem wagenartigen Rollenträger mit zwei je zwei Tastrollen 9 tragenden Achsen 11, welche über einen als Messfeder 12 wirkenden streifenförmigen Steg miteinander verbunden sind. Dieser Steg weist in seinem Mittelteil eine verdickte Lasche 13 auf, welche an der Unterseite der balkenförmigen Welle 1 befestigt ist. Der wagenartige Rollenträger 11, 12 ist also an der Welle 1 federnd gelagert.

An der Oberseite der balkenförmigen Welle 1 ist über ein Verbindungsstück 14, welches in der Art eines verstellbaren Stempels ausgebildet ist, eine zwei Führungsrollen 10 tragende Zentrierachse 15 befestigt. Das Verbindungsstück 14 ist federnd ausgebildet, so dass die Zentrierachse 15 bei einer Verschwenkung der die Tastrollen 9 tragenden Achsen 12 entsprechend gleichsinnig verstellt wird und stets alle Rollen 9 und 10 in den Nuten 8 geführt sind. Somit bildet die beschriebene dreiachsige Aufhängung eine federnde Verbindung zwischen der Welle 1 und dem Hohlzylinder 7, welche eine vertikale Verstellbewegung des Hohlzylinders 7 und damit jeder Messwalze 2 relativ zur festen Welle 1 ermöglicht. Diese Verstellbewegung, deren Amplitude nach oben durch Anschläge 16 begrenzt ist, bewirkt eine entsprechende Biegung der Messfeder 12. Wie den Fig. 4 bis 6 entnommen werden kann, liegen die Anschläge 16 in der Bewegungsbahn der Achsen 11 und wirken somit auf diese.

Bei dem in Fig. 5 dargestellten Betriebszustand befindet sich die Messwalze 2 im Ruhestand und liegt beispielsweise an der Führungsplatte 4 an, wobei das Verbindungsstück 14 so eingestellt ist, dass die Messfeder 12 nicht deformiert ist. Sobald nun ein Wickel W zwischen Führungsplatte 4 und Messwalze 2 eintritt, wird die Messwalze 2 in Abhängigkeit von der momentanen lokalen Dicke des Wickels W mit einer Kraft F nach oben gedrückt, was eine entsprechende Verbiegung der Messfeder 12 bewirkt. Dieser Betriebszustand ist in Fig. 6 dargestellt; man erkennt, dass sich die Achse des Hohlzylinders 7 um den Verstellweg a von der Mittelachse der Welle 1 nach oben verschoben hat. Durch die Biegung der Messfeder 12 entstehen in deren Material Dehnungen, welche durch auf der Messfeder 12 geeignet angeordnete Dehnungsmessstreifen in elektrische Grössen umgewandelt werden und dadurch gemessen werden können. Die Dehnungsmessstreifen sind auf die Messfeder 12 aufgeklebt oder aufgesputtet.

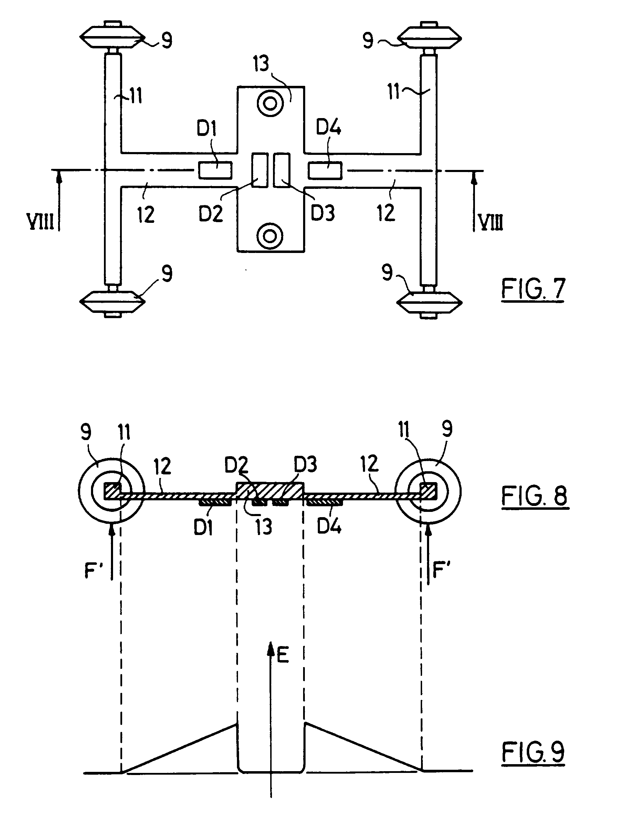

Wie insbesondere den Fig. 7 und 8 entnommen werden kann, sind auf der Messfeder 12 im Bereich der Lasche 13 zwei Dehnungsmessstreifen D2 und D3 quer zur Längsrichtung der Messfeder 12 und unmittelbar im Anschluss an die Lasche 13 je ein Dehnungsmessstreifen D1 und D4 in Längsrichtung der Messfeder 12 angeordnet. In Fig. 9 ist der Verlauf der Dehnung E, welche eine Funktion der auf die Tastrollen 9 wirkenden Kraft F' ist, über die Länge der Messfeder 12 aufgetragen. Man kann diesem Diagramm entnehmen, dass die Dehnung im Bereich der Dehnungsmessstreifen D2 und D3 gleich Null und im Bereich der Dehnungsmessstreifen D1 und D4 maximal ist. Dementsprechend werden für die Messung der Dehnung die Differenzwerte der Dehnungsmessstreifenpaare D1, D2 und D3, D4 verwendet.

Bekanntlich hat jeder Dehnungsmessstreifen D1 bis D4 einen bestimmten elektrischen Widerstand R1 bis R4, wobei diese Widerstände alle gleich gross sind. Da die relative Widerstandsänderung bei Durchbiegung der Messfeder 12 proportional zur Dehnung der Dehnungsmessstreifen ist, kann die Bestimmung der Dehnung durch eine Messung dieser Widerstandsänderung erfolgen. Dies erfolgt gemäss Fig. 10 mit einer Wheatstoneschen Brückenschaltung, welche aus vier Zweigen besteht, die von den ringförmig zusammengeschalteten Widerständen R1 bis R4 gebildet werden. Wenn man nun an die Anschlusspunkte zwischen den Widerständen R1 und R3 einerseits und R2 und R4 andererseits eine Speisespannung U anlegt, dann kann man an den beiden übrigen Anschlusspunkten eine zur Brückenverstimmung proportionale Ausgangsspannung V abgreifen, welche ihrerseits der Summe der Dehnung der einzelnen Dehnungsmessstreifen D1 bis D4 proportional ist. Da aber die Dehnung E eine Funktion der auf die Tastrollen 9 wirkenden Kraft F' und diese ihrerseits eine Funktion der auf die Messwalze 2 wirkenden Kraft F ist, ist die Ausgangsspannung V ein Mass für die Kraft F und damit für die Dicke des Wickels W.

Die Ausgangsspannung V wird an jeder der über die Breite des Wikkels hintereinander angeordneten Messwalzen gemessen, wozu von jeder Messwalze 2 entsprechende Leitungen in der balkenförmigen Welle 1 nach aussen, zu einer an einer Maschinenseite angeordneten Auswertungsstation geführt sind. Die Welle 1 mit den Messwalzen 2 erstreckt sich über die gesamte Maschinenbreite. Wenn der jeweilige Wickel W schmäler ist als diese, dann sind nur die am Wickel anliegenden Messwalzen 2 in Funktion und die Signale der nicht benötigten, inaktiven Messwalzen werden nicht abgefragt. Jede der aktiven Messwalzen 2 misst den Verlauf der Dicke des ihr zugeordneten Streifens des Wickels W über dessen Länge, und ein Vergleich der Messergebnisse der einzelnen Messwalzen 2 zu einem bestimmten Zeitpunkt liefert ein Dickenprofil über die Breite des Wickels W. Da die Messwalzen 2 in jedem Fall mit einem definierten, einstellbaren Druck von oben auf den Wickel W drücken, wirken sie gleichzeitig als Verdichtungsorgane, welche den Wickel verdichten. Dieser Verdichtungsdruck wird selbstverständlich für alle Messwalzen 2 gleich gross eingestellt. Bereichsanpassungen des Messsystems, d.h. Anpassungen an die Dicke des zu verarbeitenden Wickels W sind durch entsprechende Einstellung an der Halterung 3 (Fig. 1) einfach möglich. Das Messsystem ist somit ganz ausserordentlich flexibel und einfach an verschiedene Wickelbreiten und Wickeldicken anpassbar.

## Patentansprüche

1. Vorrichtung zum Messen der Dicke und/oder der Ungleichmässigkeit von nachfolgend als Wickel (W), bezeichneten Vliesen oder Watte an Spinnereivorbereitungsmaschinen, mit einer festen Führung für den Wickel (W) und mit einem diesen gegen die Führung drückenden und relativ zu dieser verstellbaren Dickenfühler, dessen Verstellung ein Mass für die Dicke und/oder die Ungleichmässigkeit des Wickels (W) darstellt, dadurch gekennzeichnet, dass der Dickenfühler durch eine Mehrzahl von über die Breite des Wickels (W) nebeneinander angeordneten und durch die Wickel (W) individuell verstellbaren Einzelfühlern (2) gebildet ist.

2. Vorrichtung nach Anspruch 1, dadurch gekennzeichnet, dass die Einzelfühler durch auf einer gemeinsamen Welle (1) angeordnete und relativ zu dieser verstellbare Messwalzen (2) gebildet sind.

3. Vorrichtung nach Anspruch 2, dadurch gekennzeichnet, dass die gemeinsame Welle (1) balkenförmig ausgebildet und drehfest gelagert, und dass ihr Abstand von der festen Führung (4, 5, 6, 6') einstellbar ist.

4. Vorrichtung nach Anspruch 3, dadurch gekennzeichnet, dass die feste Führung (4, 5, 6, 6') platten- oder walzenförmig ausgebildet ist.

5. Vorrichtung nach Anspruch 3 oder 4, dadurch gekennzeichnet, dass die Messwalzen (2) durch Hohlzylinder (7) gebildet und mit der gemeinsamen Welle (1) über eine federnde Aufhängung verbunden sind.

6. Vorrichtung nach Anspruch 5, dadurch gekennzeichnet, dass die federnde Aufhängung mindestens drei auf parallel zur Längsachse der gemeinsamen Welle (1) orientierten Achsen (11, 15) angeordnete Rollenpaare (9 bzw. 10) aufweist, welche einerseits an der gemeinsamen Welle federnd gelagert sind und welche anderseits die Hohlzylinder (7) an ihrer Innenwand tragen.

7. Vorrichtung nach Anspruch 6, dadurch gekennzeichnet, dass die Rollen (9, 10) in entsprechenden Nuten (8) an der Innenwand der Hohlzylinder (7) geführt sind.

8. Vorrichtung nach Anspruch 7, dadurch gekennzeichnet, dass zwei der Rollenpaare (9) tragenden Achsen (11) von einem blattfederartigen Träger (12) getragen sind, welcher senkrecht zur Achse der gemeinsamen Welle (1) und senkrecht zur Verstellrichtung der Hohlzylinder (7) angeordnet ist.

9. Vorrichtung nach Anspruch 8, dadurch gekennzeichnet, dass der blattfederartige Träger als Messfeder (12) ausgebildet und mit mindestens einem Paar von Dehnungsmessstreifen (D1, D3; D2, D4) versehen ist, wobei der eine Dehnungsmessstreifen (D1, D4) in einem Bereich von durch die Verstellung des Hohlzylinders (7) bewirkter maximaler und der andere Dehnungsmessstreifen (D2, D3) in einem Bereich von minimaler Dehnung der Messfeder angeordnet ist.

10. Vorrichtung nach Anspruch 9, dadurch gekennzeichnet, dass die gemeinsame Welle (1) einen rechteckigen Querschnitt mit zwei zur Verstellrichtung der Hohlzylinder (7) parallelen Seitenflächen aufweist, und dass die Messfeder (12) an der der Führung (3, 5, 6, 6') benachbarten Bodenfläche der gemeinsamen Welle befestigt ist.

11. Vorrichtung nach Anspruch 10, dadurch gekennzeichnet, dass die Messfeder (12) in ihrem Mittelteil eine laschenförmige Verdickung (13) aufweist und im Bereich dieser Verdickung an der gemeinsamen Welle befestigt ist, und dass die in einem Bereich minimaler Dehnung der Messfeder angeordneten Dehnungsmessstreifen (D2, D3) im Bereich dieser Verdickung vorgesehen sind.

12. Vorrichtung nach Anspruch 11, gekennzeichnet durch Anschläge (16) zur Begrenzung der Durchbiegung der Messfeder (12).

## Claims

1. Device for measuring the thickness and/or unevenness of webs or waddings, referred to hereafter as laps (W), on spinning preparation machines, with a stationary guide for the lap (W) and with a thickness sensor which presses this against the guide and is adjustable relative to the latter and the adjustment of which is a measure of the thickness and/or unevenness of the lap (W), characterised in that the thickness sensor is formed by a plurality of individual sensors (2) arranged next to one another over the width of the lap (W) and adjustable individually by the laps (W).

2. Device according to Claim 1, characterised in that the individual sensors are formed by measuring rollers (2) arranged on a common shaft (1) and adjustable relative to this.

3. Device according to Claim 2, characterised in that the common shaft (1) is made beam-shaped and is mounted fixedly in terms of rotation, and in that its distance from the stationary guide (4, 5, 6, 6') is adjustable.

4. Device according to Claim 3, characterised in that the stationary guide (4, 5, 6, 6') is made plate-shaped or roller-shaped.

5. Device according to Claim 3 or 4, characterised in that the measuring rollers (2) are formed by hollow cylinders (7) and are connected to the common shaft (1) by a resilient suspension.

6. Device according to Claim 5, characterised in that the resilient suspension has at least three pairs of rolls (9 and 10) which are arranged on axles (11, 15) oriented parallel to the longitudinal axis of the common shaft (1) and which, on the one hand, are mounted resiliently on the common shaft and which, on the other hand, carry the hollow cylinders (7) on their inner wall.

7. Device according to Claim 6, characterised in that the rolls (9, 10) are guided in corresponding grooves (8) on the inner wall of the hollow cylinders (7).

8. Device according to Claim 7, characterised in that two of the axles (11) carrying pairs of rolls (9) are carried by a support (12) which resembles a leaf spring and which is arranged perpendicularly to the axis of the common shaft (1) and perpendicularly to the direction of adjustment of the hollow cylinders (7).

9. Device according to Claim 8, characterised in that the support resembling a leaf spring is designed as a measuring spring (12) and is equipped with at least one pair of strain gauges (D1, D3; D2, D4), one strain gauge (D1, D4) being arranged in a region of maximum strain of the measuring spring brought about by the adjustment of the hollow cylinder (7), and the other strain gauge (D2, D3) being arranged in a region of minimum strain of the measuring spring.

10. Device according to Claim 9, characterised in that the common shaft (1) has a rectangular cross-section with two side faces parallel to the direction of adjustment of the hollow cylinders (7), and in that the measuring spring (12) is fastened to the bottom face of the common shaft adjacent to the guide (3, 5, 6, 6').

11. Device according to Claim 10, characterised in that the measuring spring (12) in its middle part has a bridge-shaped thickening (13) and in the region of this thickening is fastened to the common shaft, and in that the strain gauges (D2, D3) arranged in a region of minimum strain of the measuring spring are provided in the region of this thickening.

12. Device according to Claim 11, characterised by stops (16) for limiting the bending of the measuring spring (12).

## Revendications

1. Dispositif pour mesurer l'épaisseur et/ou l'irrégularité de voiles ou de nappes désignés ci-après par bobines dans des machines de préparation des métiers à filer, avec un guidage fixe pour la bobine et avec un capteur d'épaisseur poussant celle-ci contre le guidage et déplaçable relativement à celui-ci, dont le déplacement constitue une mesure de l'épaisseur et/ou de l'irrégularité de la bobine, caractérisé en ce que le capteur d'épaisseur est constitué par plusieurs capteurs individuels (2) disposés les uns à côté des autres sur la largeur de la bobine (W) et ajustables individuellement par les bobines.

2. Dispositif selon la revendication 1, caractérisé en ce que les capteurs individuels sont constitués par des rouleaux de mesure (2) disposés sur un arbre commun (1) et déplaçable relativement à celui-ci.

3. Dispositif selon la revendication 2, caractérisé en ce que l'arbre commun (1) est réalisé en forme de poutre et est logé de façon immobile en rotation, et en ce que son écart relativement au guidage fixe (4, 5, 6, 6') est réglable.

4. Dispositif selon la revendication 3, caractérisé en ce que le guidage fixe (4, 5, 6, 6') est réalisé en forme de plaque ou de rouleau.

5. Dispositif selon la revendication 3 ou 4, caractérisé en ce que les rouleaux de mesure (2) sont formés par des cylindres creux (4) et sont reliés à l'arbre commun (1) par une suspension élastique.

6. Dispositif selon la revendication 5, caractérisé en ce que la suspension élastique présente au moins trois paires de rouleau (9 et 10) disposés sur des axes (11, 15) orientés parallèlement à l'axe longitudinal de l'arbre commun (1), qui sont logés, d'une part, élastiquement à l'arbre commun et qui portent, d'autre part, les cylindres creux (7) à leur paroi intérieure.

7. Dispositif selon la revendication 6, caractérisé en ce que les rouleaux (9, 10) sont guidés dans des rainures correspondantes (8) à la paroi intérieure des cylindres creux (7).

8. Dispositif selon la revendication 7, caractérisé en ce que deux axes (11) supportant les paires de rouleau (9) sont portés par un support (12) en forme de ressort à lames qui est disposé perpendiculairement à l'axe de l'arbre commun (1) et perpendiculairement à la direction de réglage des cylindres creux (7).

9. Dispositif selon la revendication 8, caractérisé en ce que le support en forme de ressort à lames est réalisé comme ressort de mesure (12) et est pourvu d'au moins une paire de bandes de mesure de dilatation (D1, D3; D2, D4), une bande de mesure de dilatation (D1, D4) étant disposée au voisinage d'une extension maximale résultant du déplacement du cylindre creux (7) et l'autre bande de mesure de dilatation (D2, D3) au voisinage d'une extension minimale du ressort des mesure.

10. Dispositif selon la revendication 9, caractérisé en ce que l'arbre commun (1) présente une section transversale rectangulaire avec deux surfaces latérales parallèles à la direction d'ajustage des cylindres creux (7), et en ce que le ressort de mesure (12) est fixé à la surface de fond de l'arbre commun avoisinant le guidage (3, 5, 6, 6').

11. Dispositif selon la revendication 10, caractérisé en ce que le ressort de mesure (12) présente dans sa partie centrale un épaississement en forme de languette (13) et est fixé dans la zone de cet épaississement à l'arbre commun, et en ce que les bandes de mesure de dilatation (D2, D3) disposées dans une zone d'extension minimale du ressort de mesure sont prévues au voisinage de cet épaississement.

12. Dispositif selon la revendication 11, caractérisé par des butées (16) pour délimiter le fléchissement du ressort de mesure (12).
